# EUROPEAN PATENT SPECIFICATION

(11) **EP 1 803 403 B1**
(45) Date of publication and mention of the grant of the patent: **11.07.2012**
(21) Application number: 06026933.9
(22) Date of filing: 27.12.2006
(51) Int. Cl.: A61B 8/08, A61N 7/02

(54) **Ultrasound diagnostic system of detecting a lesion**
Ultraschalldiagnosesystem zum Erkennen von einer Läsion
Système diagnostique à ultrasons pour detécter une lésion

(30) Priority: 28.12.2005 KR 20050131293
(43) Date of publication of application: 04.07.2007
(73) Proprietor: SAMSUNG MEDISON CO., LTD., Kangwon-do 250-875 (KR)
(72) Inventor: Kwon, Sung Jae, Dongdaemun-gu, Seoul 130-781 (KR); Yoon, Ra Young, Gangnam-gu, Seoul 135-280 (KR)
(74) Representative: Lorenz, Markus

(56) References cited:
- WO-A1-03/075771
- WO-A1-2004/075987
- US-A- 4 574 635
- US-A1- 2005 203 399
- BEVAN P D ET AL: "B-scan ultrasound imaging of thermal coagulation in bovine liver: frequency shift attenuation mapping", ULTRASOUND IN MEDICINE AND BIOLOGY, NEW YORK, NY, US, vol. 27, no. 6, 1 June 2001 (2001-06-01), pages 809-817, XP004247076, ISSN: 0301-5629, DOI: DOI:10.1016/S0301-5629(01)00380-5

## Description

### BACKGROUND

### 1. Field

The present invention generally relates to an ultrasound diagnostic system and more particularly to an ultrasound diagnostic system for detecting a lesion using high intensity focused ultrasound.

### 2. Background

An ultrasound diagnostic system has become an important and popular diagnostic tool due to its wide range of applications. Specifically, due to its non-invasive and non-destructive nature, the ultrasound diagnostic system has been extensively used in the medical profession. Modern high-performance ultrasound diagnostic systems and techniques are commonly used to produce two or three-dimensional (2D or 3D) diagnostic images of a target object. The ultrasound diagnostic system generally uses a probe including an array transducer having a plurality of transducer elements to transmit and receive ultrasound signals. The ultrasound diagnostic system forms ultrasound images of the internal structures of the target object by electrically exciting the transducer elements to generate ultrasound pulses that travel into the target object. The ultrasound pulses produce ultrasound echoes since they are reflected from a discontinuous surface of acoustic impedance of the internal structure, which appears as discontinuities to the propagating ultrasound pulses. Various ultrasound echoes return to the array transducer and are converted into electrical signals, which are amplified and processed to produce ultrasound data for forming an image of the internal structure of the target object.

In particular, an ultrasound diagnostic system using a high intensity focused ultrasound (HIFU) to remove a lesion (e.g., malignant tumor) in a body has been known to produce excellent treatment effects in the medical field. The lesion is necrotized with heat generated by focusing the high intensity ultrasound onto the lesion so that the lesion can be removed in the body. Since the generated heat may destroy other normal tissues around of the lesion in the body, the treatment should be performed carefully while focusing the ultrasound on the lesion so as not to damage the normal tissues. Conventionally, the ultrasound diagnostic system provides an ultrasound image showing the lesion being removed by the HIFU. That is, an operator can observe the process of removing the lesion.

The HIFU significantly increases temperature of a region on which the HIFU is focused, and the necrosis begins at a focal point of the HIFU. Therefore, it is necessary to avoid focusing the HIFU on the normal tissues and to observe thermal diffusion in real time when the HIFU is injected on the lesion, so as not to damage the normal tissues. However, it is difficult to observe the thermal diffusion in the conventional ultrasound diagnostic system.

US 2005/0203399 A1 discloses a frame which ensures that the alignment between a high intensity focused ultrasound (HIFU) transducer deigned for vaginal use and a commercially available ultrasound image probe is maintained, so that the HIFU, focus remains in the image plane during HIFU therapy. A water-filled membrane placed between the HIFU transducer and the treatment site provides acoustic coupling. The coupling is evaluated to determine whether any air bubbles exist at the coupling interface, which night degrade the therapy, provided by the HIFU transducer. HIFU lesions on tissue appear as hyperechoic spots on the ultrasound image in real time during application of HIFU therapy.

WO 2004/075987 A1 discloses a high intensity focused ultrasound (HIFU) for medically treating tumors which is automatically administered under robotic control in dosage intervals that alternate with ultrasonic imaging intervals. The HIFU transmitter is re-aimed for each dosage to compensate for motion of the tumor due to heart beats and other events.

In XP004247076 B-scan attenuation mapping techniques based on the shift in center frequency of an ultrasound signal are examined. A simple technique based on the change of phase of the quadrature-demodulated signals is presented and analysed. Autoregressive analysis is also examined.

### BRIEF DESCRIPTION OF THE DRAWINGS

Arrangements and embodiments may be described in detail with reference to the following drawings in which like reference numerals refer to like elements and wherein:
FIG. 1 illustrates an ultrasound diagnostic system according to one embodiment of the present invention;
FIG. 2 illustrates a flowchart showing a process of detecting the lesion according to one embodiment of the present invention;
FIG. 3 is a graph showing two center frequencies;
FIG. 4 depicts an example of a center frequency shift image; and
FIG. 5 illustrates a flowchart showing a process of detecting the lesion according to another embodiment of the present invention.

### DETAILED DESCRIPTION

A detailed description may be provided with reference to accompanying drawings. One of ordinary skill in the art may realize that the following description is illustrative only and is not in any way limiting. Other embodiments of the present invention may readily suggest themselves to such skilled persons having the benefit of this disclosure.

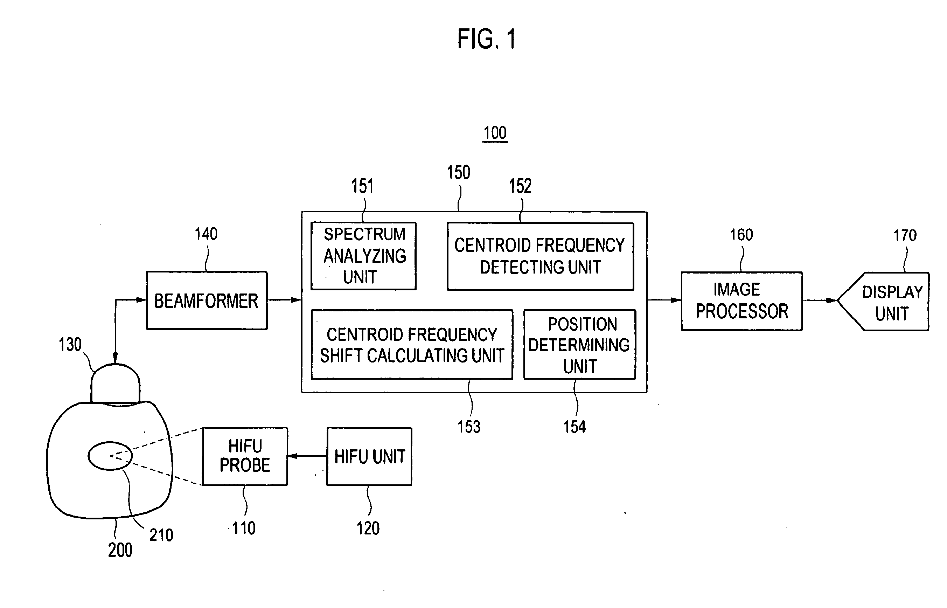

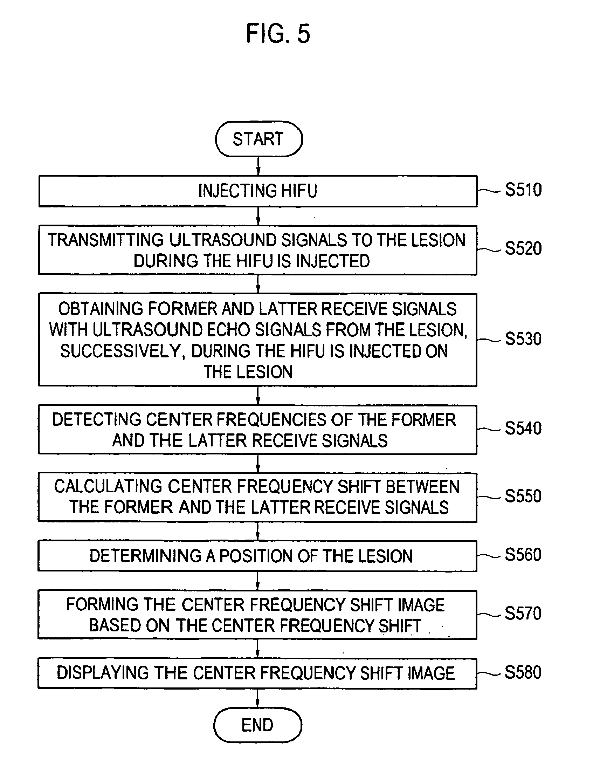

Hereinafter, embodiments of the present invention will be described with reference to FIGS. 1 and 5. The following embodiments describe detecting a lesion based on first receive signals and second receive signals. The first receive signals are obtained with ultrasound echo signals from the lesion, which has not undergone a high intensity focused ultrasound (HIFU). Further, the second receive signals are obtained with ultrasound echo signals from the lesion, which underwent the HIFU.

FIG. 1 illustrates an ultrasound diagnostic system according to one embodiment of the present invention. As shown in FIG. 1, an ultrasound diagnostic system 100 may include an HIFU probe 110, an HIFU unit 120, an imaging probe 130, a beamformer 140, a signal processing unit 150, an image processor 160 and a display unit 170. Further, the ultrasound diagnostic system 100 may include a container (not shown in FIG. 1) for containing a medium. The container may be a water tank, a water bag and the like. The container is disposed between the HIFU probe 110 and a body (i.e., skin) when the HIFU is injected into a body.

The HIFU probe 110 may inject the HIFU on a lesion 210 in a body 200. The HIFU probe 110 may include a number of elements. The HIFU unit 120 may provide the HIFU probe 110 with a high frequency power for generating the HIFU and control the HIFU probe 110 to inject the HIFU toward the lesion 210. As such, the HIFU unit 120 may include a power supply, a controller and a driver (not shown in FIG 1). The power supply provides the high frequency power to the HIFU probe 110. The controller generates driving signals, and the driver drives the HIFU probe 110 according to the control signals outputted from the controller to focus the HIFU onto the lesion 210.

The imaging probe 130 may transmit ultrasound signals to the lesion 210 in the body 200 along transmit scanlines and receive ultrasound echo signals from the lesion 210. The imaging probe 130 may further output receive signals obtained from the ultrasound echo signals for forming an ultrasound image showing the lesion 210. The imaging probe 130 may include a number of elements arranged in a form of 1-dimensional or a 2-dimensional array. The imaging probe 130 may transmit ultrasound signals to a target object, namely, the lesion 210 in the body 200. The imaging probe 130 may receive ultrasound echo signals reflected from the lesion 210 and convert the ultrasound echo signals into the first and second receive signals.

The beamformer 140 may provide appropriate delays to the ultrasound signals for transmission such that the ultrasound signals outputted from the imaging probe 130 are focused on a focal point. Further, the beamformer 140 focuses the receive signals by considering the arrival time of the echo signals at each element in the imaging probe 130.

According to an embodiment of the present invention, the signal processing unit 150 may detect the center frequencies of two receive signals outputted from the imaging probe 130 and calculate the center frequency shift between the two receive signals. The signal processing unit 150 may include a spectrum analyzing unit 151, a center frequency detecting unit 152 and a center frequency shift calculating unit 153. The spectrum analyzing unit 151 may analyze a first spectrum and a second spectrum to produce spectrum analysis results. The first spectrum and the second spectrum are obtained from the first and the second receive signals. The center frequency detecting unit 152 may detect the first and second center frequencies from the analysis results, respectively. The center frequency shift calculating unit 153 may calculate the difference between the first and second center frequencies. In other words, the center frequency shift calculating unit 153 may calculate the shift of the first center frequency of the first receive signals due to the HIFU. The center frequency difference, namely, the center frequency shift, is a pixel value of each pixel for forming a center frequency shift image.

According to another embodiment of the present invention, the signal processing unit 150 may also determine a position of the lesion in consideration of the center frequency shift. In such a case, the signal processing unit 150 may further include a position determining unit 154. The position determining unit 154 may determine a position of the lesion based on the center frequency shift. Specifically, the position determining unit 154 may search a region in which pixels having pixel values within a predetermined range are adjacent to one another based on the fact that the center frequency shift is significant at the boundary of the lesion. The boundary of the region may be adopted to determine the position of the lesion boundary.

The image processor 160 may form at least one ultrasound image showing the lesion based on the receive signals and a center frequency shift image based on the center frequency shift. Also, the image processor 160 may form an overlap image of the ultrasound image and the center frequency shift image. Thus, the image processor 160 may remove the background of the center frequency shift image to form a transparent center frequency shift image, which shows only the boundary of the lesion, to be overlapped with the ultrasound image. Specifically, in order to form the transparent center frequency shift image, the image processor 160 may set the pixel values of all the pixels outside the region to zero in the center frequency shift image. According to another embodiment, the image processor 160 may express the background of the center frequency shift image with transparency in pseudo color map so as to leave only the boundary of the lesion.

The display unit 170 may display at least one of the ultrasound image, the center frequency shift image and the overlap image. For example, by dividing a display area of the display unit 170 into a plurality of sub-areas, the ultrasound image, the center frequency shift image and the overlap image may be displayed in each respective sub-areas.

Hereinafter, a method of detecting the lesion which is not part of the present invention will be described with reference to FIGS. 1 to 4.

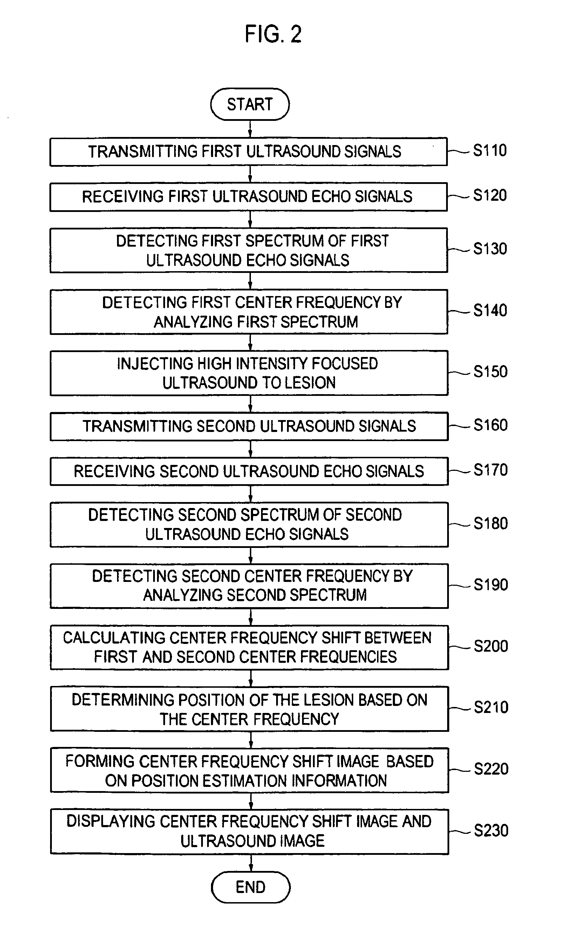

Referring now to FIGS. 1 and 2, the imaging probe 130 may transmit first ultrasound signals to a lesion in a body (step S110) and receive first ultrasound echo signals (step S120). The spectrum analyzing unit 151 may analyze the first spectrum of the first ultrasound echo signals to produce a first spectrum analysis result (step S130). Further, the center frequency detecting unit 152 may detect the first center frequency from the first spectrum based on the first spectrum analysis result (step S140).

The HIFU probe may inject the HIFU onto the lesion (step S150). Also, the imaging probe 130 may transmit second ultrasound signals to the lesion, which underwent the HIFU (step S160), and receive second ultrasound echo signals (step S170). The spectrum analyzing unit 151 may analyze a second spectrum of the second ultrasound echo signals to produce a second spectrum analysis result (step S180). The center frequency detecting unit 152 may detect a second center frequency from the second spectrum based on the second spectrum analysis result (step S190).

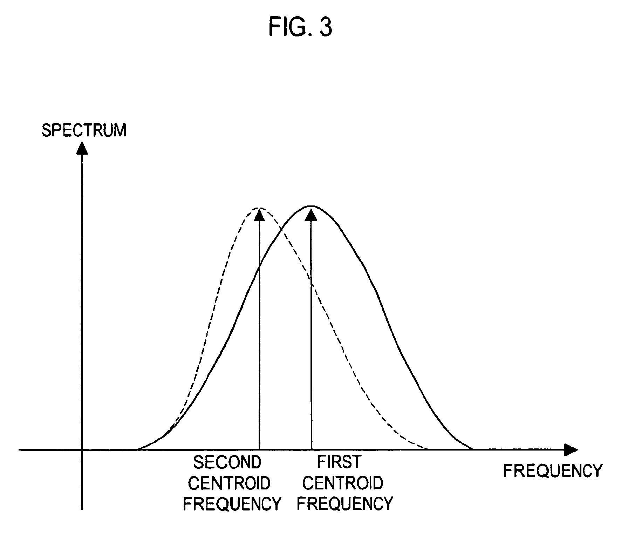

The center frequency shift calculating unit 153 may calculate a center frequency shift, that is, a difference between the first and second center frequencies as shown in FIG. 3 (step S200). The position determining unit 154 may determine the position of the lesion (i.e., boundary of the lesion) based on the center frequency shift calculated by the center frequency detecting unit 152 (step S210). The center frequency shift is a pixel value of each pixel for forming a center frequency shift image. After calculating the center frequency shift, the position determining unit 514 may search a region including pixels of which pixel values are within a predetermined range and adjacent to one another. In such a case, the position determining unit may determine the position of the lesion in consideration of a boundary of the region.

The image processor 160 may form the center frequency shift image based on the center frequency shift (step S220). The display unit 170 may display the center frequency shift image formed in the image processor 160 (step S220). In this embodiment, the steps 130 and 140 may be performed after the step 150.

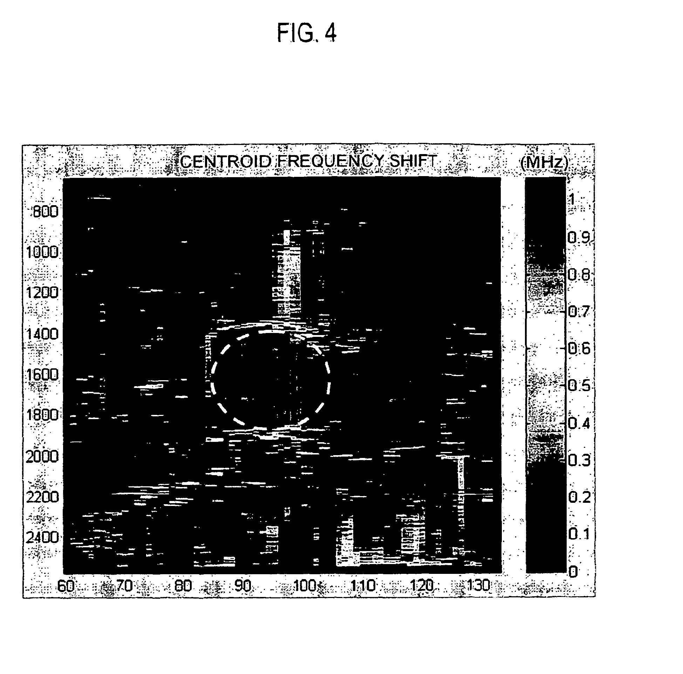

FIG. 4 depicts a center frequency shift image expressed by pixels having pixel values denoted with the central frequency shift between the first and second center frequencies. Fig. 4 shows the result based on the first and second ultrasound echo signals received at a temperature of 40°C and 70°C, respectively. In FIG. 4, the degree of the central frequency shift is denoted with a pseudo color map. In FIG. 4, a dotted circle denotes the boundary of the lesion. The dotted circle can be expressed with the determined position of the lesion boundary.

Hereinafter, a method of detecting the lesion which is not part of the present invention will be described with reference to FIGS. 1 and 5.

Referring now to FIGS. 1 and 5, the HIFU probe may inject the HIFU on a lesion 210 in a body 200 (step S510). The image probe may transmit ultrasound signals to the lesion when the HIFU is injected onto the lesion 210 (step S520) and receive former and latter ultrasound echo signals from the lesion. The image probe 130 may convert the former and latter ultrasound echo signals into former and latter receive signals (step S530). The signal processor may detect the center frequencies of the former and latter receive signals (step S540) and calculate the center frequency shift between the former and latter receive signals (step S550). The center frequency shift is a pixel value of each pixel for forming a center frequency shift image. After calculating the center frequency shift, the signal processor 150 may search a region including pixels of which pixel values are within a predetermined range and adjacent to one another. Also, the signal processor 150 may determine a position of the lesion based on the center frequency shift (step S560). In case that the signal processor searches the region, the position of the lesion may be determined in consideration of a boundary of the region. The image processor 160 may form the center frequency shift image based on the center frequency shift (step S570), while the display unit may display the center frequency shift image (step S580).

By using the embodiments, thermal diffusion due to injection of the HIFU can be detected.

In the above-described embodiment, the lesion is detected based on the difference of the center frequencies of the first and second ultrasound echo signals, which are obtained before and after the lesion undergoes the HIFU. However, in another embodiment, the lesion may be detected based on the ultrasound echo signals, which are successively obtained when the HIFU is injected onto the lesion, until the lesion is removed. In this embodiment, the center frequencies of former ultrasound receive signals and latter ultrasound receive signals are compared in order to obtain the center frequency shift.

As described above, in accordance with the present invention, the image showing the thermal diffusion due to the temperature difference generated by applying the high intensity focused ultrasound can be provided in real time to the user. Thus, the user can remove the lesion accurately while observing the thermal diffusion in real time during treatment.

An embodiment may be achieved in whole or in parts by an ultrasound diagnostic system, which includes: a high intensity frequency ultrasound (HIFU) probe for injecting the HIFU on a lesion in a body; an imaging probe for transmitting ultrasound signals to the lesion, receiving ultrasound echo signals from the lesion, and outputting receive signals obtained from the ultrasound echo signals; a signal processing unit for detecting center frequencies of two receive signals outputted from the imaging probe and calculating center frequency shift between the two receive signals; an image processor for forming at least one ultrasound image showing the lesion based on the receive signals and a center frequency shift image based on center frequency shift; and a display unit for displaying the ultrasound image and the center frequency shift image.

Any reference in this specification to "one embodiment," "an embodiment," "example embodiment," etc., means that a particular feature, structure or characteristic described in connection with the embodiment is included in at least one embodiment of the invention. The appearances of such phrases in various places in the specification are not necessarily all referring to the same embodiment. Further, when a particular feature, structure or characteristic is described in connection with any embodiment, it is submitted that it is within the purview of one skilled in the art to effect such feature, structure or characteristic in connection with other ones of the embodiments.

Although embodiments have been described with reference to a number of illustrative embodiments thereof, it should be understood that numerous other modifications and embodiments can be devised by those skilled in the art that will fall within the scope of the principles of this disclosure. More particularly, various variations and modifications are possible in the component parts and/or arrangements of the subject combination arrangement within the scope of the disclosure, the drawings and the appended claims. In addition to variations and modifications in the component parts and/or arrangements, alternative uses will also be apparent to those skilled in the art.

## Claims

1. An ultrasound diagnostic system (100), comprising:
a high intensity frequency ultrasound HIFU probe (110) for injecting the HIFU on a lesion in a body;
an imaging probe (130) for transmitting ultrasound signals to the lesion and receiving ultrasound echo signals from the lesion, the imaging probe (130) further being configured to output receive signals obtained from the ultrasound echo signals;
a signal processing unit (150) for detecting center frequencies of two receive signals outputted from the imaging probe and calculating center frequency shift between the two receive signals;
an image processor (160) for forming at least one ultrasound image showing the lesion based on the receive signals and a center frequency shift image based on the center frequency shift; and
a display unit (170) for displaying the ultrasound image and the center frequency shift image.

2. The ultrasound diagnostic system (100) of Claim 1, wherein the two receive signals include:
first receive signals obtained by the ultrasound echo signals from the lesion having not undergone the HIFU; and
second receive signals obtained by the ultrasound echo signals from the lesion having undergone the HIFU.

3. The ultrasound diagnostic system (100) of Claim 1, wherein the two receive signals include:
former receive signals and latter receive signals successively obtained when the HIFU is injected to the lesion until the lesion is removed.

4. The ultrasound diagnostic system (100) of Claim 1, wherein the signal processing unit (150) includes:
a spectrum analyzing unit (151) for analyzing spectra of the two receive signals to produce spectrum analysis results;
a center frequency detecting unit (152) for detecting center frequencies of the two receive signals, respectively, based on the spectrum analysis results; and
a center frequency shift calculating unit (153) for calculating the center frequency shift between the two receive signals.

5. The ultrasound diagnostic system (100) of Claim 1, wherein the image processor (160) forms an overlap image of the ultrasound image and the center frequency shift image, and wherein the display unit (170) displays the overlap image.

6. The ultrasound diagnostic system (100) of Claim 5, wherein the image processor (160) sets the pixel values of pixels outside a region corresponding to the lesion to zero in the center frequency shift image and forms a transparent center frequency shift image to be overlapped with the ultrasound image.

7. The ultrasound diagnostic system (100) of Claim 4, further comprising a container for containing a medium, wherein the contained medium is disposed between the HIFU probe and the body.

8. The ultrasound diagnostic system (100) of Claim 1, wherein
the signal processing unit (150) is further configured to determine a position of the lesion in consideration of the center frequency shift.

## Patentansprüche

1. Ultraschalldiagnosesystem (100), welches Folgendes aufweist:
eine Hochintensitäts-Frequenz-Ultraschall-HIFU-Sonde (110) zum Injizieren der HIFU in eine Läsion bzw. Wunde in einem Körper;
eine bildgebende Sonde (130) zum Übertragen von Ultraschallsignalen zu der Wunde und Empfangen von Ultraschallechosignalen von der Wunde, wobei die bildgebende Sonde (130) des Weiteren dafür vorgesehen ist, von den Ultraschallechosignalen erhaltene Empfangssignale auszugeben;
eine Signalverarbeitungseinrichtung (150) zum Detektieren von Mittenfrequenzen von zwei von der bildgebenden Sonde ausgegebenen Empfangssignalen und zum Berechnen einer Mittenfrequenzverschiebung zwischen den zwei Empfangssignalen;
einen Bildprozessor (160) zum Bilden weinigstens eines Ultraschallbilds, welches die Wunde basierend auf den Empfangssignalen und ein Mittenfrequenzverschiebungsbild basierend auf der Mittenfrequenzverschiebung zeigt; und
eine Anzeigeeinheit (170) zum Anzeigen des Ultraschallbilds und des Mittenfrequenzverschiebungsbilds.

2. Ultraschalldiagnosesystem (100) nach Anspruch 1, wobei die zwei Empfangssignale Folgendes aufweisen:
erste Empfangssignale, welche durch die Ultraschallechosignale von der Wunde erhalten wurden, die der HIFU nicht ausgesetzt waren; und
zweite Empfangssignale, welche durch die Ultraschallechosignale von der Wunde erhalten wurden, welche der HIFU ausgesetzt waren.

3. Ultraschalldiagnosesystem (100) nach Anspruch 1, wobei die zwei Empfangssignale Folgendes aufweisen:
weitere Empfangssignale und letztere Empfangssignale, welche aufeinanderfolgend erlangt wurden, wenn die HIFU in die Wunde injiziert wird, bis die Wunde entfernt ist.

4. Ultraschalldiagnosesystem (100) nach Anspruch 1, wobei die Signalverarbeitungseinheit (150) Folgendes aufweist:
eine Spektralanalyseeinheit (151) zum Analysieren von Spektren der zwei Empfangssignale, um Spektralanalyseergebnisse zu erzeugen;
eine Mittenfrequenzdetektiereinheit (152) zum Detektieren von Mittenfrequenzen der beiden Empfangssignale, jeweils basierend auf den Spektralanalyseergebnissen; und
eine Mittenfrequenzverschiebungs-Berechnungseinheit (153) zum Berechnen der Mittenfrequenzverschiebung zwischen den zwei Empfangssignalen.

5. Ultraschalldiagnosesystem (100) nach Anspruch 1, wobei der Bildprozessor (160) ein Überlappungsbild des Ultraschallbilds und des Mittenfrequenzverschiebungsbilds erzeugt, und wobei die Anzeigeeinheit (170) das Überlappungsbild anzeigt.

6. Ultraschalldiagnosesystem (100) nach Anspruch 5, wobei der Bildprozessor (160) die Pixelwerte von Pixeln außerhalb eines der Wunde entsprechenden Bereichs in dem Mittenfrequenzverschiebungsbild auf Null setzt und ein transparentes Mittenfrequenzverschiebungsbild erzeugt, welches dafür vorgesehen ist, mit dem Ultraschallbild überlappt zu werden.

7. Ultraschalldiagnosesystem (100) nach Anspruch 4, welches des Weiteren einen Behälter zum Aufnehmen eines Mediums aufweist, wobei das darin enthaltende Medium zwischen der HIFU-Sonde und dem Körper angeordnet ist.

8. Ultraschalldiagnosesystem (100) nach Anspruch 1, wobei die Signalverarbeitungseinheit (150) des Weiteren dafür vorgesehen ist, eine Position der Wunde unter Berücksichtigung der Mtitenfrequenzverschiebung zu ermitteln.

## Revendications

1. Système de diagnostic à ultrasons (100) comprenant :
une sonde à ultrasons focalisés de haute intensité HIFU (110) pour injecter les HIFU sur une lésion dans un corps,
une sonde d'imagerie (130) pour transmettre des signaux ultrasonores à la lésion et recevoir des signaux d'échos ultrasonores de la lésion, la sonde d'imagerie (130) étant en outre configurée pour émettre des signaux de réception obtenus à partir des signaux d'échos ultrasonores,
une unité de traitement de signaux (150) pour détecter les fréquences centrales de deux signaux de réception émis par la sonde d'imagerie et pour calculer le décalage de fréquence centrale entre les deux signaux de réception,
un processeur d'image (160) pour former au moins une image ultrasonore montrant la lésion basée sur les signaux de réception et une image de décalage de fréquence centrale basée sur le décalage de fréquence centrale, et
une unité d'affichage (170) pour afficher l'image ultrasonore et l'image de décalage de fréquence centrale.

2. Système de diagnostic à ultrasons (100) de la revendication 1, dans lequel les deux signaux de réception comprennent :
des premiers signaux de réception obtenus par les signaux d'échos ultrasonores de la lésion n'ayant pas subie les HIFU, et
des seconds signaux de réception obtenus par les signaux d'échos ultrasonores de la lésion ayant subie les HIFU.

3. Système de diagnostic à ultrasons (100) de la revendication 1, dans lequel les deux signaux de réception comprennent :
des premiers signaux de réception et des derniers signaux de réception obtenus successivement lorsque les HIFU sont injectés dans la lésion jusqu'à ce que la lésion soit enlevée.

4. Système de diagnostic à ultrasons (100) de la revendication 1, dans lequel l'unité de traitement de signaux (150) comprend :
une unité d'analyse de spectre (151) pour analyser les spectres des deux signaux de réception pour produire des résultats d'analyse de spectre,
une unité de détection de fréquence centrale (152) pour détecter respectivement les fréquences centrales des deux signaux de réception basées sur les résultats d'analyse de spectre, et
une unité de calcul du décalage de fréquence centrale (153) pour calculer le décalage de fréquence centrale entre les deux signaux de réception.

5. Système de diagnostic à ultrasons (100) de la revendication 1, dans lequel le processeur d'image (160) forme une image de chevauchement de l'image ultrasonore et de l'image de décalage de fréquence centrale, et dans lequel l'unité d'affichage (170) affiche l'image de chevauchement.

6. Système de diagnostic à ultrasons (100) de la revendication 5, dans lequel le processeur d'image (160) règle les valeurs de pixels des pixels à l'extérieur d'une région correspondant à la lésion à zéro dans l'image de décalage de fréquence centrale et forme une image de décalage de fréquence centrale transparente pour être chevauchée par l'image ultrasonore.

7. Système de diagnostic à ultrasons (100) de la revendication 4, comprenant en outre un récipient pour contenir un milieu, dans lequel le milieu contenu est disposé entre la sonde HIFU et le corps.

8. Système de diagnostic à ultrasons (100) de la revendication 1, dans lequel l'unité de traitement de signal (150) est en outre configurée pour déterminer une position de la lésion en tenant compte du décalage de fréquence centrale.
